Europäisches Patentamt

European Patent Office

Office européen des brevets

(11) Publication number: **0 172 506**
**A1**

## (12) EUROPEAN PATENT APPLICATION

(21) Application number: 85110008.1

(22) Date of filing: 08.08.85

(51) Int. Cl.⁴: **C 12 N 15/00**
C 02 F 3/34, C 12 N 1/20
//(C12R1/38, 1:40, 1:185, 1:44,
1:46, 1:07, 1:85, 1:465, 1:22,
1:41, 1:19)

(30) Priority: 14.08.84 US 640519

(43) Date of publication of application:
26.02.86 Bulletin 86/9

(84) Designated Contracting States:
AT BE CH DE FR GB IT LI LU NL SE

(71) Applicant: Occidental Chemical Corporation
360 Rainbow Boulevard South ,Box 728
Niagara Falls, N.Y. 14302(US)

(72) Inventor: Sojka, Stanley A.
160 Windsor Avenue
Buffalo, N.Y. 14209(US)

(72) Inventor: Pierce, George E.
6375 Hibiscus Court
Westerville Ohio 43081(US)

(74) Representative: Kraus, Walter, Dr. et al,
Patentanwälte Kraus, Weisert & Partner
Thomas-Wimmer-Ring 15
D-8000 München 22(DE)

(54) Cloning the halo-organic biodegradable gene.

(57) This invention relates to methods for cloning microorganisms, that are able to matabolize a broad spectrum of halogenated organic compounds to make new life forms which have specificity for metabolizing specific halogenated organic compounds in more efficient and effective manner than the progenitors. This invention also relates to the new life forms thereby produced and the application of said new life forms in processes for degrading industrial waste containing the specific halo-organic compounds desired to be disposed of.

EP 0 172 506 A1

## CLONING THE HALO-ORGANIC BIODEGRADABLE GENE

This invention relates to methods for cloning microorganisms, that are able to metabolize a broad spectrum of halogenated organic compounds to make new life forms which have specificity for metabolizing specific halogenated organic compounds in more efficient and effective manner than the progenitors. This invention also relates to the new life forms thereby produced and the application of said new life forms in processes for degrading industrial waste containing the specific halo-organic compounds desired to be disposed of.

Colaruotolo, et.al. in copending U.S. Patent Application SN 305,079 filed September 24th, 1981, which application is owned by the common assignee of this application, namely Occidental Chemical Corporation, discloses and claims new microorganisms generally identified Pseudomonas cepacia var. niagarous and mutants thereof which are useful in metabolizing a broad spectrum of halogenated organic compounds to carbon dioxide, water and a salt. Also disclosed and claimed are biologically pure plasmids identified as pRO 4.7, pRO 31, and pRO 54 contained in said Pseudonamads, which plasmids are capable of being fused with other plasmids of higher molecular weight. The Colaruotolo, et.al. application also discloses the ability of said microorganisms to metabolize a broad spectrum of halogenated organic compounds to carbon dioxide, water and a salt and their application in disposing of industrial waste containing a variety of halogenated organic compounds.

The importance of developing acceptable environmental techniques for disposing of the large volumes of mixtures of halogenated organic compound wastes, such as exists in waste landfills throughout the United States and the industrialized nations throughout the world as exemplified by the Love Canal, cannot be over emphasized. The broad spectrum biodegrading microorganisms serve a useful addition to the materials and methods available to the environmentalist to help alleviate the problems caused by such landfills. However, the need for microorganisms capable of specifically metabolizing especially recalcitrant halogenated organic compounds to innocuous materials, without creating concomitant problems is emphasized by the industrial requirements for disposing of such chemicals as Dioxins, PCB's, and the halo-organic compounds such as the chlorotoluenes.

We have now found that by employing _in vitro_ and _in vivo_ gene manipulative techniques for DNA sequences we can isolate the desirable plasmid encoded traits and make new life forms which possess an enhanced ability to biodegrade specific halo-organic compounds thereby allowing for the development of more efficient and economical processes for disposing of industrial wastes containing said specific compounds.

Among the manipulative techniques that may be employed in accordance with our invention and in order to make new genetic materials having an enhanced ability to biodegrade specific halo-organic compounds are the following:

1) Cloning of restriction enzyme generated plasmid-DNA fragments into _E. Coli_ type microorganisms, i.e., using the restriction/ligation procedure described by Cohen et.al. (see Cohen et.al. 1972. _Proc. Nat. Acad. Sci._ _70_: 1293-1297) and the transformation procedure described by Kushner. (See Kushner, S.R. 1978. An Improved Method for Transformation of _Escherichia coli_ with ColEl Derived Plasmids, in, Proceedings of the International Symposium on Genetic Engineering. Elsevier/North-Holland Biomedical Press.)

2) Cloning plasmid-DNA from two different microorganisms using the Cohen et.al. restriction/ligation procedure, followed by Kushner transformation and then a three way conjugation as described by Ditta et.al. (see Ditta, G., S. Stanfield, D. Corbin, and D. R. Helinski, 1980. Broad host range DNA cloning system for gram-negative bacteria: Construction of a gene bank of _Rhizobium meliloti_. _Proc. Nat. Acad. Sci._ 77:7347-7351.)

More particularly, a preferred method to be employed in accordance with our invention comprises:

The process for producing a microorganism having specificity for

- 3 -

biodegradable halo-organics which comprises **0172506**

   a) separately culturing and maintaining 1) a broad spectrum microorganism selected from the group of ATCC 31945, ATCC 31941, ATCC 31942, ATCC 31940, ATCC 31943, ATCC 31944, ATCC 31939,and mutants thereof, and 2) a broad host range vector,

   b) separately isolating the plasmid-DNA from (1) and (2) above,

   c) separately purifying the plasmid-DNA from (1) and (2) above,

   d) separately enzymatically restricting the purified DNA from (1) and (2) above,

   e) combining the products of step (d) and enzymatically ligating the combined products,

   f) transforming the products of the ligation, into a receptive microorganism such as _E. Coli_ or _Pseudomonas_ spp,

   g) selecting those transformants having the desired plasmid-DNA inserted into the vector, from step (e) and (f),

   h) conjugating the selected plasmid-DNA into a receptive host selected from the group of _Pseudomonas_, _Klebsiella Rhizobium_, _Agrobacterium_, _Escherichia_ with aid of a helper plasmid, and

   i) selecting those transconjugants having the desired plasmid-DNA.

The preferred method to be employed in accordance with our invention is best understood by describing it with respect to specific Examples however it is to be understood that our methods may be applied generally to the materials embraced within the scope of our claimed invention. The microorganisms ATCC 31939 through ATCC 31945 are also known as (a/k/a) other names as given in the following Table I, which lists their synonyms, and are available from the American Type Culture Collection, Rockville, MD by virtue of their disclosure and claims in the Colaruotolo U.S. SN 305,079 which has been published by the European

- 4 -

Patent Office as Publication Number 0,075,885 on April 6th, 1983 in Bulletin 83/14.

### Table I - Synonyms

| ATCC 31945 | a/k/a | HCI | (2CT) | a/k/a | H-1 |
|---|---|---|---|---|---|
| ATCC 31941 | a/k/a | HCIV | (3CT) | a/k/a | H-3 |
| ATCC 31942 | a/k/a | HCV | (2,4DCB) | a/k/a | H-2 |
| ATCC 31940 | a/k/a | HCV | (3,4DCB) | a/k/a | H-4 |
| ATCC 31943 | a/k/a | HCV | (2,6DCT)-2 | a/k/a | SS-3 |
| ATCC 31944 | a/k/a | HCV | (2,6DCT)-3 | a/k/a | H-6 |
| ATCC 31939 | a/k/a | HCV | (3,4DCT)-5 | a/k/a | H-5 |

These and other materials described herein are all useful as starting materials for making the new recombinant plasmids in accordance with our invention and for their transformation and transconjugation into a variety of microbial hosts.

### EXAMPLE I

#### Isolation of Plasmid-DNA

Five (5) ml of medium, namely Luria broth supplemented with 10 mg/ml of tetracycline for $\underline{E. coli}$ HB101 (pVK100) and Tn medium (Vanderbergh, et.al. 1981) supplemented with 150 ppm of 3,4-dichlorotoluene (Pierce, et.al. 1983) for ATCC 31939, was inoculated with $\underline{E. coli}$ HB101 (pVK100) and incubated at 35-37°C overnight and with ATCC 31939 and incubated at 25-27°C overnight, respectively. Plasmid isolation from $\underline{E.}$ coli HB104 (pVK100) and ATCC 31939 were conducted separately as follows, according to the Horowitz modification of the Birnbolm and Doly (1979) technique. After growth overnight, as indicated above, 1.5 ml of culture was poured into an Eppendorf tube and centrifuged for 1 minute in an Eppendorf centrifuge. The medium was removed by aspiration, leaving the pellet as dry as possible. The

- 5 -

pellet was resuspended in 100 ul of an ice-cold solution of: 50mM glucose, 10mM EDTA, 25mM Tris.HCl (pH 8.0) and 4 mg/ml lysozyme. The resuspended pellet was stored at room temperature for 5 minutes and then 200ul of a freshly-prepared ice-cold solution of: 0.2N NaOH and 1.0% SDS was added. The contents of the tube were then mixed by inverting rapidly, 2 to 3 times. The tube was then stored on ice for 5 minutes. To the contents of the tube, 150ul of an ice-cold solution of potassium acetate (approximately pH 4.8) was added such that the final solution is now 3M with respect to potassium and 5M with respect to acetate. The contents of the tube were then mixed gently by inversion and then stored on ice for 5 minutes. The tube was then centrifuged for 5 minutes in an Eppendorf centrifuge and the supernatant, was then transferred to a fresh Eppendorf tube; an equal volume of phenol/chloroform was added and the tube was mixed by vortexing and then centrifuged for 2 minutes in an Eppendorf centrifuge. Again the supernatant was transferred to a fresh Eppendorf tube. Two volumes of ethanol, were added to the supernatant and allowed to stand at room temperature for 2 minutes. The tube was then centrifuged, at room temperature, in an Eppendorf centrifuge for 5 minutes. The supernatant was removed and the tube is inverted on a paper towel and the remaining fluid was allowed to drain off. To the tube 1 ml of 70% ethanol is then added. The tube was then vortexed briefly and then centrifuged for 5 minutes in an Eppendorf centrifuge. The supernatant is again removed and the pellet was dried briefly in a vacuum dessicator. To the tube 50ul of a solution of pH 8.0 TE buffer containing 20 mg/ml of DNase-free pancreatic RNase was added and the tube was vortexed briefly. The plasmid-DNA can now be subjected to restriction.

Where required Pseudomonad plasmid-DNA was further purified by cesium chloride isopycnic ultracentrifugation, namely the DNA pellet was dissolved in 3.8ml of TE buffer and stored overnight at 4°C. After overnight storage 4.2g of cesium chloride was added (to give 1.55-1.56g of cesium chloride per ml), and 250ul of a 10mg/ml solution of ethidium bromide was then added. The mixture was then spun at room temperature for 10 minutes at 10,000 rpm in a Beckman J2-21 centrifuge. The supernatant was transferred to a Beckman heat seal tube and centrifuged for 48 hours at 40,000 rpm (using a Beckman $T_I$-80 rotor) in a Beckman

- 6 -

L-9 ultracentrifuge. The plasmid-DNA band was then removed with a 25 ga. needle, extracted 4 to 8 times with equal volumes of isopropanol and then dialyzed against 4 changes of TE buffer for 24 hours. The plasmid-DNA was then precipitated with ethanol to concentrate and then dissolved in TE buffer.

## EXAMPLE II
### Restriction and Ligation

The plasmid-DNA preparations obtained from E. coli HB101 (pVK100 and ATCC 31939 were then separately restricted as follows. Restriction endonuclease Hind III (obtained from BRL) was used according to the specifications supplied by BRL. Restrictions with Hind III were conducted at 37°C for 1-2 hours and then heated at 70°C for 10 minutes to inactive the restriction enzyme.

Ligation of the Hind III generated fragments of plasmid-DNA from E. coli HB101 (pVK100) and ATCC 31939 was performed with the aid of $T_4$-DNA-ligase (obtained from New England Biolabs) as follows. In a 1.5 ml Eppendorf tube, ligations were conducted according to the specifications supplied by New England Biolabs such that the total reaction volume was betwen 10-20ul and that the insert-DNA (namely, Hind III generated fragments of plasmid-DNA from ATCC 31939) was at a 2 molar excess to the vector (namely, pVK100) DNA. The ligation was carried out at 16°C for 16-20 hours. The ligation was terminated by heating the reaction mixture at 70°C for 10 minutes.

The hybrid plasmids (pVK100:ATCC 31939) constructed by the ligation were then transformed into a suitable host microorganism.

EXAMPLE III

Transformation

Prior to transformation the host to be transformed, namely E. coli AC-80 was prepared as follows. A culture of E. coli AC-80 was inoculated onto Luria-agar and allowed to grow overnight at 35-37°C. From this overnight growth, large individual colonies were used to inoculate 10 ml volumes of Luria broth which were then incubated at 37°C for 2-3 hours, (until a cell density of 5.0 x $10^7$ cfu/ml was achieved).

Cells of E. coli AC-80 so prepared were then transformed with the hybrid-plasmid-DNA mentioned previously, according to the procedure of Kushner (1978) and as modified by Pierce, et.al. (in press), as follows. 1 x $10^8$ cells of host, namely E. coli AC-80, were added to a 15 ml Corex tube and centrifuged 10 minutes at 8000 rpm. The supernatant was immediately poured off and the pellet resuspended gently with 1 ml of a 10mM solution of MOPS (pH 7.0) buffer containing 10mM RbCl. This mixture was then immediately centrifuged again for 10 minutes at 8000 rpm. The supernatant was then poured off and the tube was drained thoroughly by inverting on a paper towel. The pellet was then gently resuspended in 0.2 ml of the above buffer. To this mixture 3.0ml of DMSO and 0.2 ng of plasmid-DNA, namely (pVK100:Hind III ATCC 31339) was added. This mixture was then incubated for 40 minutes on ice. The mixture was then heat shocked at 43.5°C for 45 seconds. The volume of the mixture was then brought up to 5 ml with Z broth (pH 7.5) and allowed to outgrow for 40 minutes at 37°C prior to plating on Luria agar supplemented with 10 ug/ml tetracycline. Transformants were selected by their inability to grow on Luria agar supplemented with Kanamycin because of insertional activation of the Kanamycin resistance gene in the vector, namely pVK100, by the inserted plasmid-DNA, from namely the Hind III generated fragments of ATCC 31339 plasmid-DNA.

Transformants, namely E. coli AC-80 containing the vector pVK100 which itself contains inserts of Hind-III generated plasmid-DNA from ATCC 31339, were characterized by gel electrophoresis.

- 8 -

Individual transformants, namely E. coli AC-80 (pVK100:Hind-III ATCC 31339-TC4), were then mobilized with the aid of helper plasmid, namely pRK2013, into a receptive host, namely Pseudomonas putida 2440.

## EXAMPLE IV
### Three-Way Conjugal Cross

Matings were performed by mixing $10^9$ cells each of donor, helper and host, namely E. coli AC-80 (pVK100-Hind-III ATCC 31339-TC4), E. coli CSR603 (pRK 2013) and P. putida 2440 respectively, and then filtering the suspension onto 0.45um filters (obtained from Millipore). The filters were then incubated at 30°C on non-selective medium for 3-6 hours before plating on selective medium. All colonies which grew on King's medium supplemented with tetracycline were presumptive transconjugants, namely P. putida 2440 (pVK100: Hind-III ATCC 39339). The transconjugant named and others so constructed were then verified by gel electrophoresis prior to ascertaining their hydrocarbon utilization spectrum.

The work done herein was all in conformity with physical and biological containment requirements specified in the guidelines published by the National Institute of Health, Washington, D.C., United States of America.

The new life forms of this invention are deposited with In Vitro International, Inc., 7885 Jackson Road, Ann Arbor, Michigan, 48103, phone 1-800-IN VITRO which is an approved international depository authority under the Budapest Treaty on the International Recognition of the Deposit of Microorganisms for the purposes of Patent Protection (see WIPO, Budapest Notification #34 dated November 3, 1983).

In accordance with the foregoing procedures, the following new life forms identified in Table II were prepared and deposited with In Vitro International and have the following identification and accession numbers:

## TABLE II - NEW LIFE FORMS

| NUMBER | GROUP IDENTIFICATION | TRANSFORMANTS NOMENCLATURE | IVI ACCESSION NUMBER |
|---|---|---|---|
| 1 | TF-4 | E. coli AC-80 [pVK100:Hind-III-H5-TF4] | IVI-10012 |
| 2 | TF-11 | E. coli AC-80 [pVK100:Hind-III-H5-TF11] | IVI-10013 |
| 3 | TF-19 | E. coli AC-80 [pVK100:Hind-III-H5-TF19] | IVI-10014 |
| 4 | TF-20 | E. coli AC-80 [pVK100:Hind-III-H5 TF20] | IVI-10015 |
| 5 | TF-30 | E. coli AC-80 [pVK100:Hind-III-H5-TF30] | IVI-10016 |

| NUMBER | GROUP IDENTIFICATION | TRANSCONJUGANTS NOMENCLATURE | IVI ACCESSION NUMBER |
|---|---|---|---|
| 6 | TC-1 | P. putida 2440 [pVK100:Hind-III-H5-TF4]TC-1 | IVI-10017 |
| 7 | TC-3 | P. putida 2440 [pVK100:Hind-III-H5-TF4]TC-3 | IVI-10018 |
| 8 | TC-13 | P. putida 2440 [pVK100:Hind-III-H5-TF11]TC-13 | IVI-10019 |
| 9 | TC-22 | P. putida 2440 [pVK100:Hind-III-H5-TF19]TC-22 | IVI-10020 |
| 10 | TC-32 | P. putida 2440 [pVK100:Hind-III-H5-TF20]TC-32 | IVI-10021 |
| 11 | TC-35 | P. putida 2440 [pVK100:Hind-III-H5-TF20]TC-35 | IVI-10022 |
| 12 | TC-37 | P. putida 2440 [pVK100:Hind-III-H5-TF20]TC-37 | IVI-10023 |
| 13 | TC-40 | P. putida 2440 [pVK100:Hind-III-H5-TF20]TC-40 | IVI-10024 |
| 14 | TC-41 | P. putida 2440 [pVK100:Hind-III-H5-TF20]TC-41 | IVI-10025 |
| 15 | TC-42 | P. putida 2440 [pVK100:Hind-III-H5-TF20]TC-42 | IVI-10026 |
| 16 | TC-48 | P. putida 2440 [pVK100:Hind-III-H5-TF30]TC-48 | IVI-10027 |

The microorganisms described above have the utilization characteristics on the following carbon sources as given in the following Table III which shows the growth of the 11 transconjugants on the compounds listed at 15 days. To minimize confusion, only growth which was 1/2 level higher or more from the mean is shown. (Therefore if the mean growth on a compound was 2, only those transconjugants which had a growth of 2+ or higher would be recorded on the Table.) A note of caution must be mentioned regarding these data presented in the Table below because the medium employed also contained levels of antibiotics which would preclude the growth of the new host P. putida 2440. It is quite possible that for substrates which require complex induction the antibiotics present in the medium might exhibit sublethal effects and thus delay or retard growth. However, for this evaluation, previous experience dictates that for recent genetic constructs antibiotic pressure may be necessary to maintain the hybrid plasmid.

The transconjugants have an additional value because their host P. putida has a growth optimum at 30°C as opposed to an optimum of 26°C for the progenitor strain.

- 11 -

## TABLE III

### SUPERIOR UTILIZATION OF SELECTED CARBON SOURCES BY TRANSCONJUGANTS (a,b,c)

| SOLE CARBON SOURCE | TRANSCONJUGANTS | | | | | | | | | | | HOST P. putida |
| --- | --- | --- | --- | --- | --- | --- | --- | --- | --- | --- | --- | --- |
| | 6 | 7 | 8 | 9 | 10 | 11 | 12 | 13 | 14 | 15 | 16 | 2440 |
| Sodium Benzoate | | 4/ | | | | | | | 4/4 | | | NG (d) |
| Lindane | 1+/1+ | 1+/1+ | | | | | | | | | | NG |
| Tetrachloroethane | 2/2 | 1+/2 | /2 | /2 | 2/2 | | /2 | 2/2 | 2/2 | 2/2 | 2/2 | NG |
| Ethylenedichloride | 2/2 | 2/2 | | | 2/2 | 2/2 | 2/2 | 2/2 | 2/2 | 2/2 | 2/2 | NG |
| Methylchloroform | 2/2 | 2/2 | 2/2 | 2/2 | 2/2 | | /2 | 2/2 | | /2 | 2/2 | NG |
| Chloroform | /2 | | /2 | 2/2 | 2/2 | 2/2 | /2 | 2/2 | | 2/2 | 2/2 | NG |
| Trichloroethylene | 2/2 | 2/2 | 2/ | 2/ | 2/2 | 2/ | 2/2 | 2/2 | | | 2/2 | NG |
| 2,4-D | /2 | /2 | | | /2 | /2 | | /2 | /2 | | | NG |
| 3,4-dichlorobenzoate | | 2/2 | | /2 | 2/2 | | 2/2 | | | 2/ | 2/2 | NG |
| 2,4-dichlorobenzoate | 2/2 | 2/2 | /2 | 2/2 | 2/ | 2/ | 2/ | 2/ | | 2/ | 2/ | |

a) Results are recorded for 300/100 ppm of substrate.

b) Tetracycline at 10mg/ml was incorporated into the evaluation media.

c) Only growth which was 1/2 better than the mean is recorded.

d) NG = no growth recorded.

From a review of the results on utilization of the new life forms made in accordance with our invention, and much to our surprise, the new transconjugants have superior utilization for specific aliphatic halogenated organic compounds such as tetrachloroethane, ethylene dichloride, methylchloroform and trichloroethylene, whereas, their progenitors ATCC 31939-31945 have utilization for a broad spectrum of halogenated aromatic organic compounds as disclosed in the Colaruotolo et.al. application SN 305,079. Accordingly, our invention provides the ability to tailor a microorganism capable of biodegrading specific halogenated aliphatic organic compounds from the plasmid-DNA taken from microorganism which primarily degrade halogenated aromatic compounds.

It should also be noted that there is a wide latitude of hosts that the plasmid-DNA of this invention can be inserted into. Accordingly, we can fine tune the placement of the DNA into microorganisms so that they are in harmony with the environment that they are to be used in. For example, if the temperature of the environment they are to be employed in is elevated above the normal temperature then a host such as Pseudomonas Aeruginosa should be used, or, if low temperature is involved, then Pseudomonas Fluorescens should be used as the host. Other environmental conditions such as pH, oxygen availability can also be accomodated by selection of the host.

Among the genera of microorganisms which may be employed as hosts for the recombinant plasmid-DNA of this invention by transformation are the, <u>Genera</u> <u>Pseudonomas</u>, <u>Escherichia</u>, <u>Staphyloccus</u>, <u>Streptococcus</u>, <u>Bacillus</u>, <u>Saccaromyces</u>, <u>Stereptomyces</u>, <u>Neurospora</u>. Selection of the hosts to be employed if of course determined by the environment, and the conditions prevailing as well as, the ultimate biodegradation of the targeted organic halogenated compounds involved.

Among the genera of microorganisms which may be employed as hosts for the recombinant plasmids-DNA of this invention by transformation are the <u>Genera</u> <u>Pseudomonas</u>, <u>Klebsiella</u>, <u>Rhizobium</u>, <u>Agrobacterium</u>, <u>Escherichia</u>, among others, some of these may require the use of helper plasmids, and special vectors. Because the plasmid-DNA of this invention

- 13 -

0172506

may be inserted in a variety of hosts, a number of options become available for selecting the final host for inserting the recombinant plasmid-DNA, of this invention Accordingly, it is contemplated, in the accordance with this invention that the plasmid-DNA claimed may be inserted in anaerobic bacteria such as <u>Methanobacterium</u>, <u>Methansarcina</u>, <u>Acetobacterium</u>, <u>Butyribacterium</u>, <u>Desulfurvibrio</u>, <u>Clostridium</u>, <u>Eubacterium</u>, <u>Bifidobacterium</u>, <u>Lactobacillus</u>, among others.

Furthermore, this invention contemplates inserting the recombinant plasmid-DNA of this invention, which is capable of biodegrading specific halogenated organic compounds, into plant cells by employing a suitable vector, to allow for use of halogenated organic compounds, as pesticides for those plants especially the halogenated organic pesticides which have such an efficient and effective control of pests, whether they be insects or weeds, for which they have been designed. Accordingly, in accordance with this invention, a plant which has built-in resistance and biodegradating ability for halogenated organic pesticides is allowed for.

The new life forms of this invention may be employed in the biodegradation of the halogenated organic compounds for which they have been prepared to biodegrade, in any one of a number of application techniques. For example, new life forms may be formulated into stabilized compositions with or without nutrients and other additives, and may be employed in outdoor environments, in open water applications, or a seed to be dispersed on landfills either on dry or damp soil. Since the new life forms may act aeroborically or anareobically they may be employed on a surface of soils to be decontaminated or mixed into depths of soil where the amount of air is negligible. The new life forms of this invention may also be employed in single or multi-tank closed installation wastewater treatment systems, and this of course is an advantage since the containment of the halogenated organics is readily controllable in such systems. Our new life forms may be used in various wastewater treatment processes whether continuous, batch or sequential batch reactors. The new life forms of this invention are also especially adapted to be used as inoculants for activated sludge in

- 14 -

those processes where the disposal of the halogenated organics is involved in sewage or wastewater treatment systems either municipal or industrial including effluents and leachates from landfills such as the Love Canal. Various other techniques may be employed in the use of the new life forms of this invention for the purpose of biodegrading the specific halogenated organic compounds for which they have been designed. Although our invention has been described using specific examples and certain preferred embodiments thereof, we do not intend that our invention be limited in scope except as expressly defined in the appended claims.

We claim:

## CLAIM 1

A recombinant plasmid adapted for transformation of a microbial host said plasmid comprising a plasmid vector into which DNA has been inserted which encodes for the biodegradation of halogenated organic compounds.

## CLAIM 2 A microorganism which includes a recombinant plasmid of Claim 1.

## CLAIM 3 A transconjugant microorganism which includes a recombinant plasmid of Claim 1.

## CLAIM 4 A transconjugant of Claim 3 wherein the microorganisms is P. putida.

## CLAIM 5 A transformant microorganism of Claim 2 wherein the microorganism is selected from the group consisting of the Genera Pseudonomas, Escherichia, Staphyloccus, Streptococcus, Bacillus, Saccaromyces, Stereptomyces, Neurospora.

## CLAIM 6 A transformant of Claim 2 selected from the group IVI-10012, IVI-10013, IVI-10014, IVI-10015, IVI-10016 and mutants thereof.

## CLAIM 7 A transformant of Claim 6 namely IVI-10012 and mutants thereof.

## CLAIM 8 A transformant of Claim 6 namely IVI-10013 and mutants thereof.

## CLAIM 9 A transformant of Claim 6 namely IVI-10014 and mutants thereof.

## CLAIM 10 A transformant of Claim 6 namely IVI-10015 and mutants thereof.

## CLAIM 11 A transformant of Claim 6 namely IVI-10016 and mutants thereof.

## CLAIM 12 A transconjugant of Claim 3 selected from the group IVI-10017, IVI-10018, IVI-10019, IVI-10020, IVI-10021, IVI-10022, IVI-10023, IVI-10024, IVI-10025, IVI-10026, IVI-10027 and mutants thereof.

## CLAIM 13 A transconjugant of Claim 12 namely IVI-10017 and mutants thereof.

## CLAIM 14 A transconjugant of Claim 12 namely IVI-10018 and mutants thereof.

## CLAIM 15 A transconjugant of Claim 12 namely IVI-10019 and mutants thereof.

## CLAIM 16 A transconjugant of Claim 12 namely IVI-10020 and mutants thereof.

## CLAIM 17 A transconjugant of Claim 12 namely IVI-10021 and mutants thereof.

## CLAIM 18 A transconjugant of Claim 12 namely IVI-10022 and mutants thereof.

## CLAIM 19 A transconjugant of Claim 12 namely IVI-10023 and mutants thereof.

## CLAIM 20 A transconjugant of Claim 12 namely IVI-10024 and mutants thereof.

## CLAIM 21 A transconjugant of Claim 12 namely IVI-10025 and mutants thereof.

## CLAIM 22 A transconjugant of Claim 12 namely IVI-10026 and mutants thereof.

## CLAIM 23 A transconjugant of Claim 12 namely IVI-10027 and mutants thereof.

## CLAIM 24 The process for producing a microorganism having specificity for biodegradable halogenated organic compounds which comprises:

a) separately culturing and maintaining 1) a broad spectrum microorganism selected from the group of ATCC 31945, ATCC 31941, ATCC 31942, ATCC 31940, ATCC 31943, ATCC 31944, ATCC 31939 and mutants thereof, and 2) a broad host range vector,

b) separately isolating the plasmid-DNA from (1) and (2) above,

c) separately purifying the plasmid-DNA from (1) and (2) above,

d) separately enzymatically restricting the purified DNA from (1) and (2) above,

e) combining the products of step (d) and enzymatically ligating the combined products,

f) transforming the products of the ligation, into a receptive microorganism such as E. coli or Pseudomonas. spp.

CLAIM 25 The process of Claim 24 comprising:

g) selecting those transformants having the desired
plasmid-DNA inserted into the vector, and

h) conjugating the selected plasmid-DNA into a receptive
host selected from the group of <u>Pseudomonas</u>, <u>Klebsiella</u>
<u>Rhizobium</u>, <u>Agrobacterium</u>, <u>Escherichia</u> with aid of a
helper plasmid, and

i) selecting those transconjugants having the desired
plasmid DNA.

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| D,A | EP - A1 - 0 075 885 (OCCIDENTAL CHEMICAL CORPORATION)<br>  * Claims 1-8,11,24-28 *<br>  -- | 1-5, 24,25 | C 12 N 15/00<br>C 02 F 3/34<br>C 12 N 1/20<br>//C 12 R 1:38<br>C 12 R 1:40<br>C 12 R 1:185<br>C 12 R 1:44<br>C 12 R 1:46<br>C 12 R 1:07<br>C 12 R 1:85<br>C 12 R 1:465<br>C 12 R 1:22<br>C 12 R 1:41<br>C 12 R 1:19 |
| A | EP - A2 - 0 076 953 (MICROLIFE GENETICS, INC.)<br>  * Claim 1 *<br>  ---- | 1,2 | |
| | | | **TECHNICAL FIELDS SEARCHED (Int. Cl.4)**<br><br>C 12 N<br>C 02 F |

The present search report has been drawn up for all claims

| Place of search | Date of completion of the search | Examiner |
|---|---|---|
| VIENNA | 04-11-1985 | WOLF |